# EUROPEAN PATENT APPLICATION

(11) **EP 2 479 577 A1**
(43) Date of publication of application: **25.07.2012**
(21) Application number: 10817295.8
(22) Date of filing: 17.09.2010
(51) Int. Cl.: G01N 33/66, G01N 33/574

(54) **HEPATOCELLULAR CARCINOMA MARKER**

(30) Priority: 18.09.2009 JP 2009217586
(71) Applicant: Mitsubishi Chemical Corporation, Chiyoda-ku Tokyo 100-8251 (JP); National University Corporation Kagawa University, Takamatsu-shi, Kagawa 760-8521 (JP); Inter-University Research Institute Corporation National Institutes of Natural Sciences, Mitaka-shi Tokyo 181 8588 (JP)
(72) Inventor: IKENAKA, Kazuhiro, Okazaki-shi, Aichi 4448787 (JP); DEGUCHI, Akihiro, Kita-gun,Kawaga 7610793 (JP); HIGASHI, Mikito, Kanagawa 2278502 (JP)
(74) Representative: Jappy, John William Graham
(86) International application number: PCT/JP2010/066244
(87) International publication number: WO 2011/034182

(57) **Abstract**

A hepatocellular carcinoma marker comprising a sugar chain represented by any one of the (1) to (7) shown below is used for a method for diagnosis of hepatocellular carcinoma, especially for differential diagnosis between liver cirrhosis and hepatocellular carcinoma.

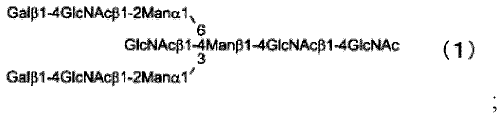

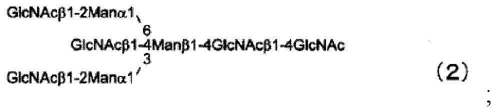

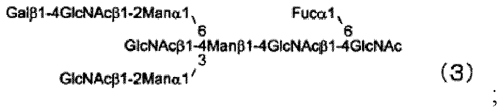

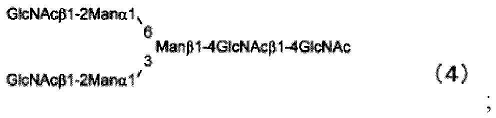

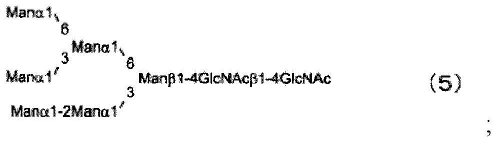

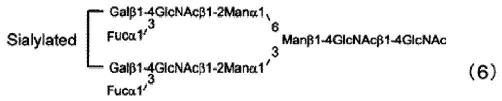

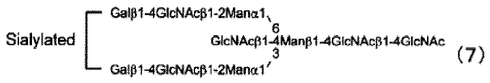

## Description

### TECHNICAL FIELD

The present invention relates to a hepatocellular carcinoma marker to be used for diagnosis of hepatocellular carcinoma in a subject animal, and a method of differential diagnosis between liver cirrhosis and hepatocellular carcinoma using the marker.

### BACKGROUND ART

In recent years, the fact that many proteins have sugar chains bound thereto has become widely known, and it is becoming clear that sugar chains bound to proteins play roles as signals for adhesion or separation between cells, and that they also act as cofactors involved in synthesis, transportation, degradation and the like of proteins.
It has been reported that even the existence of only a single sugar in a complicated sugar chain structure may change a function of the protein to which the sugar chain is bound. For example, immunoglobulins, which exist in the serum in large amounts, have binding regions for N-linked sugar chains in their Fc region. It has been reported that not less than 100 times of difference in the antibody-dependent cytotoxicity exists between immunoglobulins having, among sugar chain structures which are bound to the above binding regions, a sugar chain structure in which fucose is attached to GlcNAc at the reducing end and immunoglobulins having a sugar chain structure in which fucose is not attached thereto
However, since purification of a glycoprotein sugar chain requires enormous time and a systematic analysis method has not been developed yet, simple, sensitive and quantitative analysis of glycoprotein sugar chains has been impossible.

Under such circumstances, Ikenaka, one of the present inventors, and collaborators discovered by a method using an HPLC system that Triantennary trigalactosylated structure with one outer arm fucosylation (hereinafter referred to as "A3G3Fo"), which is a sugar chain, was increased in the sera of patients suffering from lung cancer (Non-patent Documents 1 and 2). Further, Patent Document 1 discloses a cancer detection method wherein lung cancer is detected based on A3G3Fo, and a cancer detection substance to be used in the method.
However, in the above-described Patent Document and Non-patent Documents, only glycoprotein sugar chains especially specific to lung cancer are disclosed, and glycoprotein sugar chains in cancers of other tissues are not disclosed.

It is known that, since the liver synthesizes almost all of the glycoproteins in the blood, expression of glycoproteins in the serum fluctuates when inflammation occurred in the body or when the liver itself was damaged. α-fetoprotein (AFP) and protein induced vitamin K II (PIVKAII) are hepatocellular carcinoma markers which have been clinically used so far, and these markers increase not only in the cases of hepatocellular carcinoma but also in the cases of inflammatory reaction. Further, these markers often do not increase in the cases of early-stage hepatocellular carcinoma. Thus, these markers were insufficient in terms of accuracy and specificity of detection of hepatocellular carcinoma. Under such circumstances, in terms of AFP, it is known that diagnostic accuracy (specificity) for hepatocellular carcinoma can be increased by measuring AFP having a fucosylated sugar chain, but no marker exists at present which enables accurate differential diagnosis between liver cirrhosis and hepatocellular carcinoma.

Based on a series of recent studies, it has been reported that, in the serum and progenitor cells of hepatocytes of patients suffering from hepatocellular carcinoma, the activities of glycosyltransferases, which synthesize sugar chains, are increased, and sugar chain structures which are not found in normal mature hepatocytes are expressed. However, no molecule has been discovered which can be used as an index for accurate differential diagnosis between liver cirrhosis and hepatocellular carcinoma.
Under such a situation, a method based on analysis of abnormality of glycoprotein sugar chains in the serum of patients suffering from liver diseases, which method enables to distinguish between liver cirrhosis and hepatocellular carcinoma and allows accurate detection of early-stage hepatocellular carcinoma, has been demanded.

### PRIOR ART DOCUMENTS

### Patent Document

Patent Document 1: JP 2001-289860 A

### Non-patent Documents

Non-patent Document 1: J. Biochem. 129, 537-542, 2001
Non-patent Document 2: Anal. Biochem. 267, 336-343, 1996

### SUMMARY OF THE INVENTION

The present invention aims to provide a biomarker for a method of diagnosis of hepatocellular carcinoma, especially for differential diagnosis between liver cirrhosis and hepatocellular carcinoma, in a subject animal; and to provide a method of diagnosis of hepatocellular carcinoma, and the like using the marker.

The present inventors intensively studied to solve the above-described problems and measured the amounts of biantennary N-linked sugar chains, especially neutral sugar chains M5A, A2G0, A2G0B, A2G1(6)FB and A2G2B; and asialo sugar chains A2G2B and A2G2Fo2; in the sera of subjects, and discovered that the amounts of those sugar chains contained in the sera of patients suffering from hepatocellular carcinoma can be clearly distinguished from the amounts of those sugar chains contained in the sera of healthy individuals or patients suffering from liver cirrhosis. Further, the present inventors discovered that, by using as indices the amounts of the neutral sugar chains M5, A2G0B, A2G1(6)FB and A2G2B in the sera of subjects, patients suffering from early-stage hepatocellular carcinoma which is diagnosed as Stage I according to the TNM classification can be detected, and differential diagnosis between liver cirrhosis and hepatocellular carcinoma is possible. The present inventors also discovered that, by using these hepatocellular carcinoma markers, early-stage hepatocellular carcinoma, which cannot be detected with existing hepatocellular carcinoma markers AFP and PIVKAII, can be detected, thereby completed the present invention.

The present invention provides the followings.
[1] A hepatocellular carcinoma marker comprising a sugar chain represented by any one of the (1) to (7) shown below: 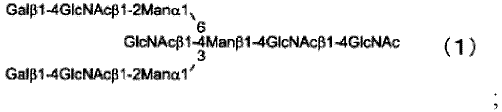 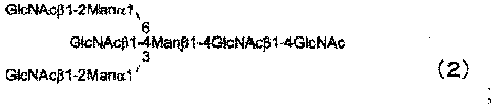 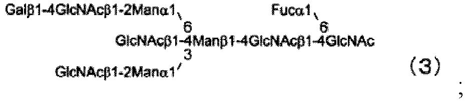 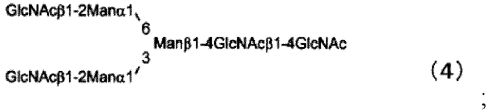 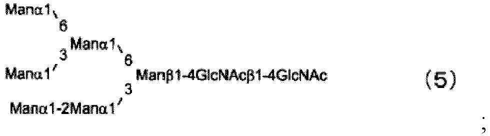 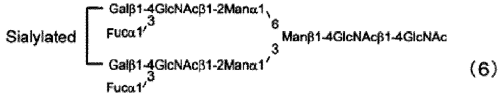 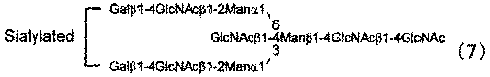
[2] A measurement method for diagnosis of hepatocellular carcinoma in a subject animal, comprising measuring the amount of the hepatocellular carcinoma marker according to [1] in body fluid collected from said animal, and using the amount of the hepatocellular carcinoma marker or a value calculated based on the amount of the hepatocellular carcinoma marker as an index.
[3] The measurement method for diagnosis of hepatocellular carcinoma according to [2], wherein said step of measuring the amount of said hepatocellular carcinoma marker according to [1] comprises preliminarily extracting a complex of a protein which is bound to the sugar chain as the marker and the sugar chain from the body fluid by using a substance which specifically binds to the protein.
[4] A method for evaluating a prophylactic or therapeutic effect against hepatocellular carcinoma in an animal in need of prophylaxis or treatment of hepatocellular carcinoma, comprising
   administering a prophylactic or therapeutic agent for hepatocellular carcinoma to said animal,
   measuring the amount of said hepatocellular carcinoma marker according to [1] in body fluid collected from said animal, and
   using the amount of said hepatocellular carcinoma marker or a value calculated based on the amount of said hepatocellular carcinoma marker as an index.
[5] The method for evaluating a prophylactic or therapeutic effect against hepatocellular carcinoma according to [4], wherein said step of measuring the amount of said hepatocellular carcinoma marker according to [1] comprises preliminarily extracting a complex of a protein which is bound to the sugar chain as the marker and the sugar chain from the body fluid by using a substance which specifically binds to the protein.
[6] A method for evaluating a prophylactic or therapeutic effect of a candidate drug compound against hepatocellular carcinoma, comprising
   administering a candidate drug compound to an animal in need of prophylaxis or treatment of hepatocellular carcinoma,
   measuring the amount of said hepatocellular carcinoma marker according to [1] in body fluid collected from said animal, and
   using the amount of said hepatocellular carcinoma marker or a value calculated based on the amount of said hepatocellular carcinoma marker as an index.
[7] The method for evaluating a prophylactic or therapeutic effect of a candidate drug compound according to [6], wherein said step of measuring the amount of said hepatocellular carcinoma marker according to [1] comprises preliminarily extracting a complex of a protein which is bound to the sugar chain as the marker and the sugar chain from the body fluid by using a substance which specifically binds to the protein.
[8] The method according to any of [2] to [7], wherein said value calculated based on the amount of said hepatocellular carcinoma marker is a value calculated using the ratio between the amounts of: a sugar chain showing no significant difference in the content between the samples from healthy animals and the samples from animals suffering from hepatocellular carcinoma; and said hepatocellular carcinoma marker.
[9] A kit for diagnosis of hepatocellular carcinoma, for evaluation of a therapeutic effect against hepatocellular carcinoma, or for evaluation of a prophylactic or therapeutic effect of a candidate drug compound against hepatocellular carcinoma, said kit comprising a reagent with which the amount of any of said hepatocellular carcinoma markers according to [1] can be measured.

The hepatocellular carcinoma marker provided by the present invention is effective for clearer diagnosis hepatocellular carcinoma because the abundance of the marker in the body fluid is clearly different between patients suffering from hepatocellular carcinoma and healthy individuals, and also different between patients suffering from hepatocellular carcinoma and patient suffering from liver cirrhosis.

### BRIEF DESCRIPTION OF THE DRAWINGS

Fig. 1 is a two-dimensional map of standard sugar chains.
Fig. 2-1 is a diagram wherein the abundance values of a hepatocellular carcinoma marker of the present invention (A2G0B) in the sera of patients suffering from various diseases are plotted.
Fig. 2-2 is a diagram wherein the abundance values of a hepatocellular carcinoma marker of the present invention (A2G0) in the sera of patients suffering from various diseases are plotted.

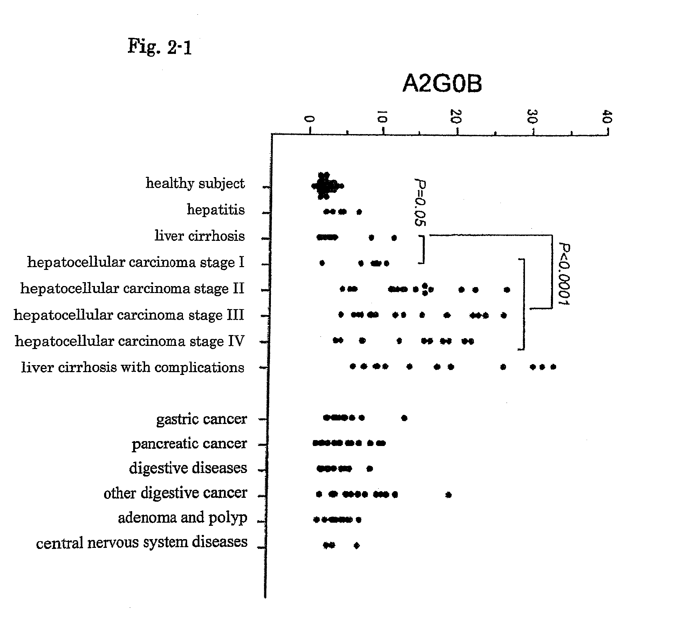

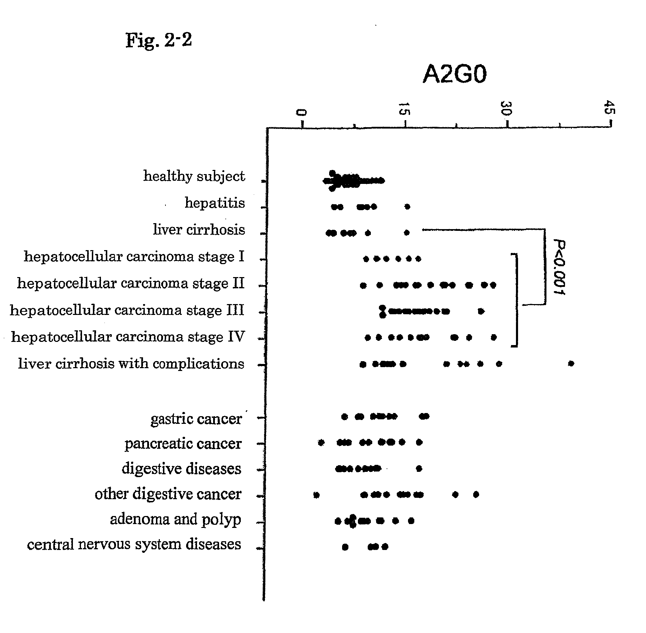

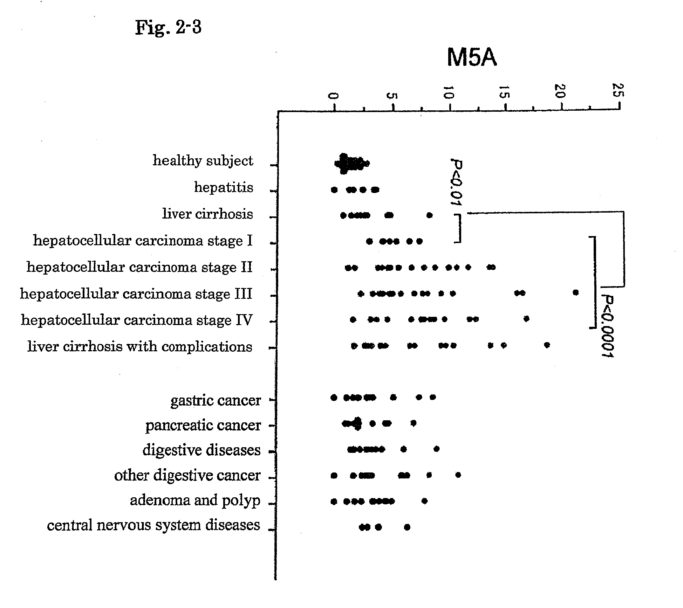

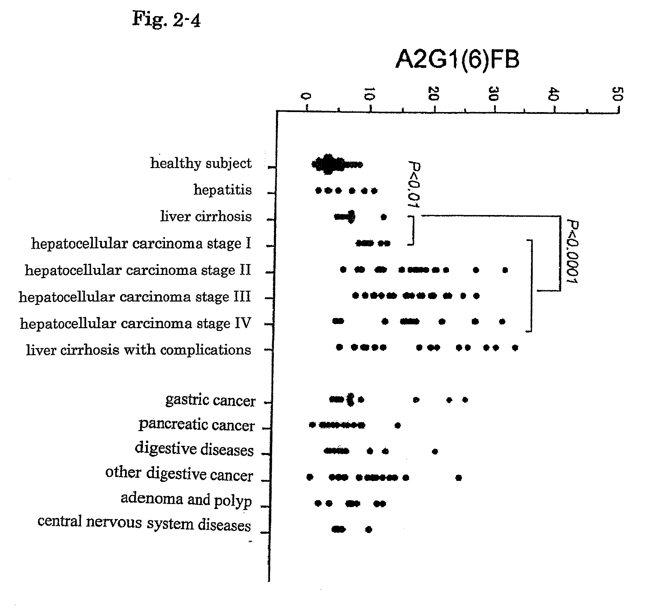

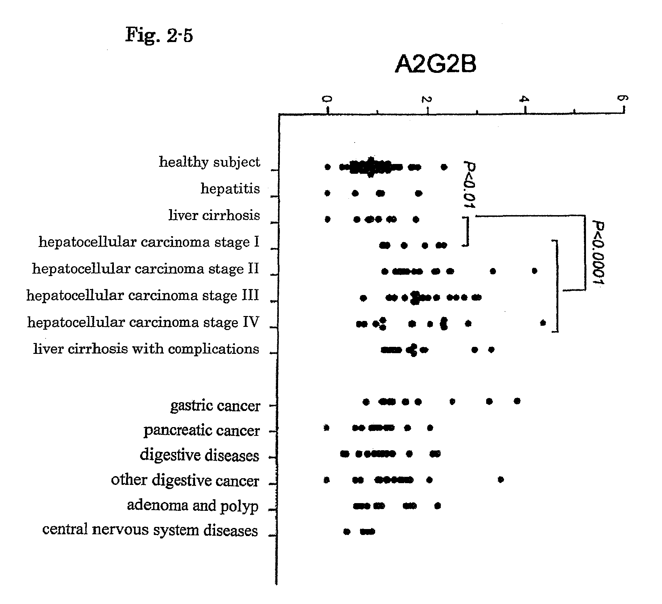

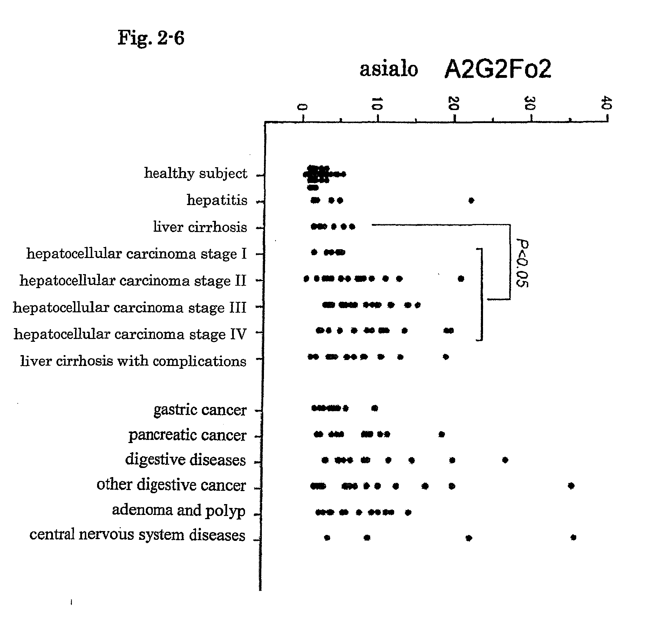

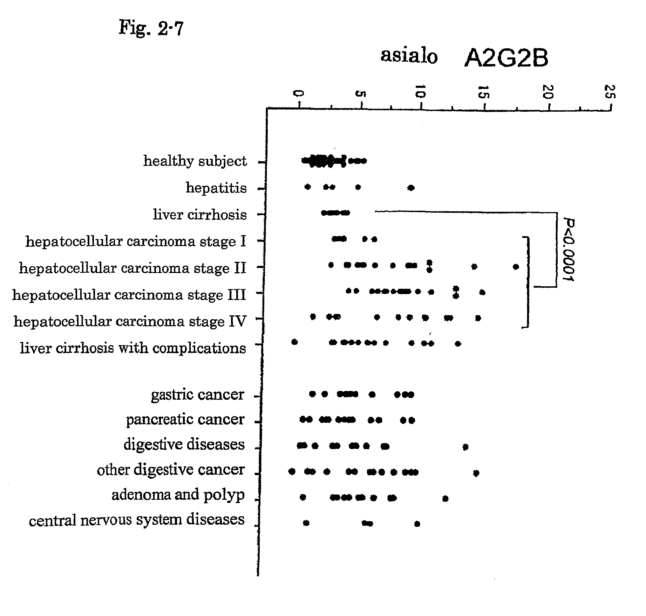

Fig. 2-3 is a diagram wherein the abundance values of a hepatocellular carcinoma marker of the present invention (M5A) in the sera of patients suffering from various diseases are plotted.
Fig. 2-4 is a diagram wherein the abundance values of a hepatocellular carcinoma marker of the present invention (A2G1(6)FB) in the sera of patients suffering from various diseases are plotted.
Fig. 2-5 is a diagram wherein the abundance values of a hepatocellular carcinoma marker of the present invention (A2G2B) in the sera of patients suffering from various diseases are plotted.
Fig. 2-6 is a diagram wherein the abundance values of a hepatocellular carcinoma marker of the present invention (asialo A2G2Fo2) in the sera of patients suffering from various diseases are plotted.
Fig. 2-7 is a diagram wherein the abundance values of a hepatocellular carcinoma marker of the present invention (asialo A2G2B) in the sera of patients suffering from various diseases are plotted.

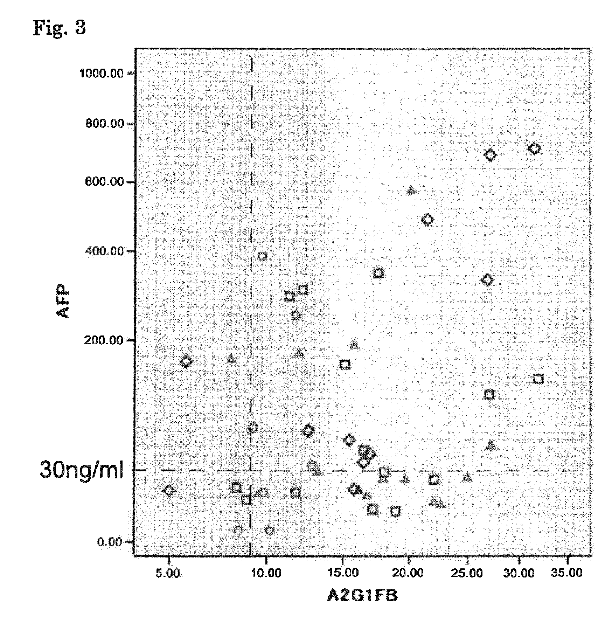

Fig. 3 is a diagram wherein medical test values for AFP (ordinate) and for A2G1(6)FB (abscissa) in hepatocellular carcinoma samples are plotted.

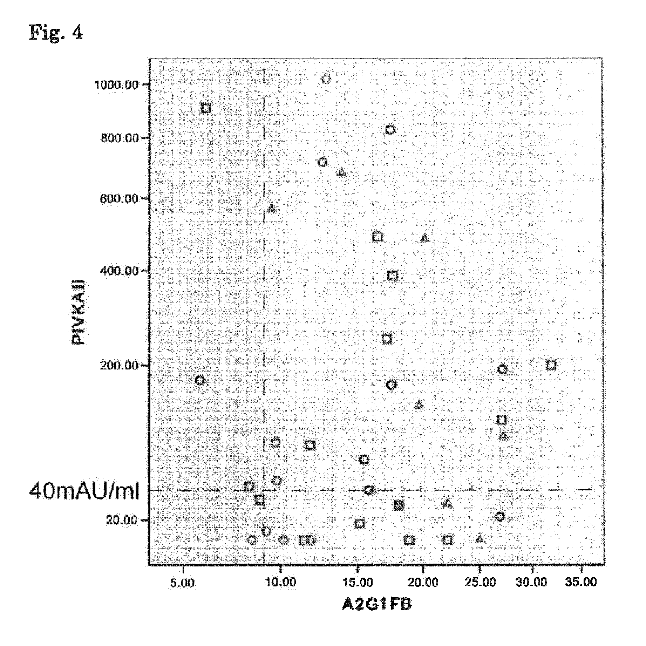

Fig. 4 is a diagram wherein medical test values for PIVKAII (ordinate) and for A2G1(6)FB (abscissa) in hepatocellular carcinoma samples are plotted.

### EMBODIMENTS FOR CARRYING OUT THE INVENTION

### <Hepatocellular Carcinoma Marker>

The hepatocellular carcinoma marker of the present invention at least comprises a sugar chain represented by any one of the Chemical Structural Formulae (1) to (7) described above.
The sugar chain shown in (1) may be hereinafter referred to as "A2G2B".
The sugar chain shown in (2) may be hereinafter referred to as "A2G0B".
The sugar chain shown in (3) may be hereinafter referred to as "A2G1(6)FB".
The sugar chain shown in (4) may be hereinafter referred to as "A2G0".
The sugar chain shown in (5) may be hereinafter referred to as "M5A".
The sugar chain shown in (6) may be hereinafter referred to as "sialylated A2G2Fo2". This sugar chain has sialic acid as a side chain in the serum, but the asialo type (this may be hereinafter referred to as "asialo A2G2Fo2"), which is prepared by deletion of the sialic acid by a known method, is also included therein.
The sugar chain shown in (7) may be hereinafter referred to as "sialylated A2G2B". This sugar chain also has sialic acid as a side chain in the serum, but the asialo type (this may be hereinafter referred to as "asialo A2G2B"), which is prepared by deletion of the sialic acid by a known method, is also included therein.

The hepatocellular carcinoma markers of the present invention are sugar chain moieties of glycoproteins contained in the sera of patients suffering from hepatocellular carcinoma in large amounts, and, in addition to the above-described sugar chains, those to which a peptide is bound are also included in the hepatocellular carcinoma markers of the present invention.

### <Method for Diagnosis of Hepatocellular Carcinoma>

The present invention also include a method for diagnosis of hepatocellular carcinoma, wherein the amount of the hepatocellular carcinoma marker is measured, and the amount of the hepatocellular carcinoma marker or a value calculated based on the amount of the hepatocellular carcinoma marker is used as an index. Examples of the sample to be used in this measurement method include body fluids collected from subject animals which may be suffering from hepatocellular carcinoma. Examples of the body fluid to be used include blood, lymph, spinal fluid and urine, and processed products thereof. The body fluid to be used is preferably blood, more preferably serum obtained by subjecting the blood to separation. In terms of the subject of the method for diagnosis of hepatocellular carcinoma, the subject animal is preferably human.

In order to measure the content of a sugar chain as the hepatocellular carcinoma marker of the present invention (this may be hereinafter referred to as a "marker sugar chain") in the sample, the marker sugar chain contained in the sample needs to be isolated from the protein to which the marker sugar chain is bound. Examples of the method for isolating the marker sugar chain from the protein include per se known methods which are normally used, more particular examples of the method include hydrazinolysis and enzyme (N-glycanase) digestion. Among these, hydrazinolysis is preferred for quantitative cleavage of a sugar chain, and, for example, the method described in Y. Otake et al., J Biochem (Tokyo) 129 (2001) 537-42 or the like is preferably employed. Here, in cases where hydrazinolysis is employed, reacetylation of the acetyl group eliminated by the hydrazinolysis is necessary. More particularly, for example, the method described in K. Tanabe et al., Anal. Biochem. 348 (2006) 324-6 or the like is employed. Further, in the case of a sialylated sugar chain, a sialic acid-cleaving enzyme such as neuraminidase may be used to cleave sialic acid, followed by purification by ion-exchange chromatography or the like.
Before the isolation of the marker sugar chain from the protein, a substance, more particularly, an antibody or the like, which specifically binds to a protein bound to the marker sugar chain may be used to extract a complex comprising the protein and the sugar chain from body fluid obtained from the sample, which is preferred in view of efficiency of the subsequent structural analysis of the sugar chain. The extraction using a substance which specifically binds to the protein may be carried out by a per se known method which is normally used. Examples of the protein which is bound to the marker sugar chain of the present invention include those described in Table 1.

**[Table 1]**

| **Gene ID** | **NCBI_No** | | **Name** |
|---|---|---|---|
| 5004 | AAI43315 | A1AG1_HUMAN | Alpha-1-acid glycoprotein 1 |
| 5005 | NP_000599 | A1AG2_HUMAN | Alpha-1-acid glycoprotein 2 |
| 5265 | AAB59495 | A1AT_HUMAN | Alpha-1-antitrypsin |
| 1 | AAH35719 | A1BG_HUMAN | Alpha-1B-glycoprotein |
| 197 | NP_001613 | FETUA_HUMAN | Alpha-2-HS-glycoprotein |
| 2 | EAW88590 | A2MG_HUMAN | Alpha-2-macroglobulin |
| 462 | CAA48690 | ANT3_HUMAN | Antithiombin-III |
| 336 | AAD34604 | APOA1_HUMAN | Apolipoprotein A-I |
| 337 | NP_000473 | APOA4_HUMAN | Apolipoprotein A-IV |
| 338 | AAA53373 | APOB_HUMAN | Apolipoprotein B-100 |
| 347 | AAB35919 | APOD_HUMAN | Apolipoprotein D |
| 51742 | | ARI4B_HUMAN | AT-rich interactive domain-containing protein 4B |
| 350 | AAA51766 | APOH_HUMAN | Beta-2-glycoprotein 1 |
| 722 | NP_000706 | C4BPA_HUMAN | C4b-binding protein alpha chain |
| 1356 | BAA08084 | CERU_HUMAN | Ceruloplasmin |
| 1191 | AAH10514 | CLUS_HUMAN | Clusterin |
| 718 | NP_000055 | CO3_HUMAN | Complement C3 |
| 720 | AAA51855 | CO4A_HUMAN | Complement C4-A |
| 721 | AAA59651 | C04B_HUMAN | Complement C4-B |
| 735 | NP_001728 | C09_HUMAN | Complement component C9 |
| 629 | AAH07990 | CFAB_HUMAN | Complement factor B |
| 3075 | CAA68704 | CFAH_HUMAN | Complement factor H |
| 866 | NP_001747 | CBG_HUMAN | Corticosteroid-binding globulin |
| 60412 | AAH26174 | EXOC4_HUMAN | Exocyst complex component 4 |
| 2335 | AAI43764 | FINC_HUMAN | Fibronectin |
| 3959 | NP_005558 | LG3BP_HUMAN | Galectin-3-binding protein |
| 2934 | AAH26033 | GELS_HUMAN | Gelsolin |
| 3240 | AAG01170 | HPT_HUMAN | Haptoglobin |
| 3250 | AAA88081 | HPTR_HUMAN | Haptoglobin-related protein |
| 3263 | AAA58678 | HEMO_HUMAN | Hemopexin |
| 3053 | NP_000176 | HEP2_HUMAN | Heparin cofactor 2 |
| 3273 | AAH69574 | HRG_HUMAN | Histidine-rich glycoprotein |
| 58508 | AAK00583 | MLL3_HUMAN | Histone-lysine N-methyltransferase MLL3 |
| 3493 | AAA52735 | IGHA1_HUMAN | Ig alpha-1 chain C region |
| 3494 | AAB59396 | IGHA2_HUMAN | Ig alpha-2 chain C region |
| 3500 | AAA70227 | IGHG1_HUMAN | Ig gamma-1 chain C region |
| 3501 | AAN76044 | IGHG2_HUMAN | Ig gamma-2 chain C region |
| 3502 | AAA70228 | IGHG3_HUMAN | Ig gamma-3 chain C region |
| 3503 | AAB59394 | IGHG4_HUMAN | Ig gamma-4 chain C region |
| 3514 | AAA58921 | IGKC_HUMAN | Ig kappa chain C region |
| 3507 | AAA52824 | IGHM_HUMAN | Ig mu chain C region |
| 3512 | NP_653247 | IGJ_HUMAN | Immunoglobulin J chain |
| 57523 | NP_079357 | K1305_HUMAN | Integrase catalytic domain-containing protein KIAA1305 |
| 3697 | CAA49279 | ITIH1_HUMAN | Inter-alpha-trypsin inhibitor heavy chain H1 |
| 3698 | NP_002207 | ITIH2_HUMAN | Inter-alpha-trypsin inhibitor heavy chain H2 |
| 3700 | AAI36394 | ITIH4_HUMAN | Inter-alpha-trypsin inhibitor heavy chain H4 |
| 3827 | AAH26253 | KNG1_HUMAN | Kininogen-1 |
| 4060 | AAH35997 | LUM_HUMAN | Lumican |
| 114770 | NP_443122 | PGRP2_HUMAN | N-acetylmuramoyl-L-alanine amidase |
| 5176 | AAA60058 | PEDF_HUMAN | Pigment epithelium-derived factor |
| 3818 | AAI17352 | KLKB1_HUMAN | Plasma kallikrein |
| 710 | NP_001027466 | IC1_HUMAN | Plasma protease C1 inhibitor |
| 5340 | AAA36451 | PLMN_HUMAN | Plasminogen |
| 5473 | AAK29642 | CXCL7_HUMAN | Platelet basic protein |
| 11107 | NP_061169 | PRDM5_HUMAN | PR domain zinc finger protein 5 |
| 5858 | CAA38255 | PZP_HUMAN | Pregnancy zone protein |
| 26091 | AAH39600 | HERC4_HUMAN | Probable E3 ubiquitin-protein ligase HERC4 |
| 259 | AAA59196 | AMBP_HUMAN | Protein AMBP |
| 2147 | AAI06931 | THRB_HUMAN | Prothrombin |
| 157807 | AAI01378 | RLBL2_HUMAN | Retinaldehyde-binding protein 1-like protein 2 |
| 7018 | NP_001054 | TRFE_HUMAN | Serotransferrin |
| 5444 | AAH74719 | PON1_HUMAN | Serum paraoxonase/arylesterase 1 |
| 7276 | ABI63345 | TTHY_HUMAN | Transthyretin |
| 2638 | AAA61704 | VTDB_HUMAN | Vitamin D-binding protein |
| 7448 | AAH05046 | VTNC_HUMAN | Vitronectin |
| 563 | NP_001176 | ZA2G_HUMAN | Zinc-alpha-2-glycoprotein |

The thus isolated marker sugar chain is labeled as required. The method of labeling is not restricted, and, in cases where a mass spectrograph is used, TMAPA (trimethyl(4-aminophenyl)ammonium chloride), which enhances the ionization efficiency, is especially preferred, and, in cases where a fluorescence detector is used, 2-aminopyridine is preferred. In terms of the method of derivatization, in the case of TMAPA, the method described in M. Okamoto et al., Rapid Commun Mass Spectrom 9 (1995) 641-3, or the like is used, and, in the case of 2-aminopyridine, the method described in Y. Otake et al., J Biochem (Tokyo) 129 (2001) 537-42, or the like is used.

The method for detecting, or for measuring the content of, a sugar chain as the hepatocellular carcinoma marker is not restricted as long as the marker sugar chain of the present invention can be detected thereby, and examples of the method include normal-phase and reversed-phase high-performance liquid chromatography, mass spectrometry, nuclear magnetic resonance, and methods using an antibody or lectin specific to the marker sugar chain of the present invention. The sugar chain as a hepatocellular carcinoma marker of the present invention is an N-linked sugar chain, and hence can be detected using a method of simple, highly accurate and quantitative detection of a bisecting-type or Lewis X-type sugar chain structure, which is a N-linked sugar chain. In the present invention, the bisecting-type sugar chain structure means a biantennary N-linked sugar chain structure in which a Man residue of the trimannosyl core structure has GlcNAc bound to the 4-position. In the present invention, the Lewis X-type sugar chain structure means an N-linked sugar chain structure in which the GlcNAc residue at the non-reducing end has fucose at the 3-position.

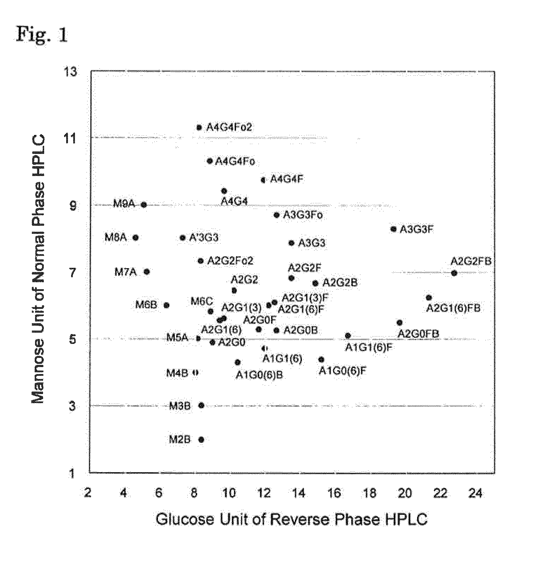

Since isolation of the marker sugar chain of the present invention from similar sugar chains existing in the serum is very difficult, it is preferred to use a detection/measurement method by the combination of liquid chromatography and a mass spectrometer (this may be hereinafter referred to as the "LC-MS method"), or to use a detection/measurement method by liquid chromatography. The structure of the sugar chain may be analyzed by any method, and, more particularly, the structure is preferably identified by, for example, a process wherein commercially available mannose standard sugar chains are subjected to normal-phase (or reversed-phase) liquid chromatography (hereinafter referred to as "normal-phase HPLC") to calculate mannose standard sugar chain peaks based on an internal standard, and commercially available galactose standard sugar chains are subj ected to reversed-phase (or normal-phase) liquid chromatography (hereinafter referred to as "reversed-phase HPLC") to calculate galactose standard sugar chain peaks based on an internal standard, to prepare a two-dimensional map (e.g., the one shown in Fig. 1), which is then used for the identification.

Examples of the method for measuring the marker sugar chain of the present invention using the LC-MS method include the method described below in detail. The specification of the liquid chromatography is not restricted as long as the liquid chromatography can stably send a liquid. The ionization method may be either ESI or APCI, and ESI is most preferred. The mass spectrometer may be any of the quadrupole type, TOF type, ion trap type, magnetic field type and Fourier transform type, among which the quadrupole type, which is highly quantitative, and the TOF type and the ion trap type, which are highly sensitive, are especially preferred.

The column to be used may be any of the normal-phase type, reversed-phase type and absorption type as long as a plurality of types of sugar chains can be separated from each other with the column. The column is preferably a reversed-phase column C8, C18 or C30, among which a C30 column is more preferred. The size of the column is not restricted, and preferably not more than 2.1 mm in terms of the inner diameter in order to reduce the flow rate and to thereby increase the sensitivity. The size is especially preferably not more than 1.5 mm.

The eluent is appropriately selected depending on the properties of the column, and, in cases where a C30 column is used, for example, 5 mM aqueous ammonium solution (pH=4) is used as the eluent A, and a mixture of 90% 5 mM aqueous ammonium acetate solution (pH=4) and 10% acetonitrile is used as the eluent B. In this case, sufficient equilibration with 0% to 30% B solution (= 100% to 70% A solution; description of the concentration of the A solution is hereinafter omitted) is necessary before injection of a sample. The concentration of the B solution upon the equilibration is preferably 10 to 25%, especially preferably 15 to 20%.

About 20 µL of a sample solution is injected to the column, and, immediately after the injection, the eluent composition is made to linearly change from about 20% B solution to about 50% B solution for about 50 minutes. The composition may be kept to be 50% B solution from minute 50 to minute 60, but the condition is not restricted since the gradient condition is limited by the type of the column. The measurement conditions for the mass spectrometry are not restricted as long as the conditions are within the ranges in which the marker sugar chain of the present invention can be separated, and excellent detection is possible under the conditions of, for example, a capillary voltage (ionization voltage) of 4000 V, nebulizer gas pressure of 45 psi, dry gas flow rate of 10 L/min. and temperature of 350°C.

The detection method is most preferably SIM (Selective Ion Monitoring) in cases where the mass spectrometer is the quadrupole type, and, in such cases, the ion to be detected is set to an ion of any of the marker sugar chains of the present invention or, when another sugar chain is used as an internal standard, the ion to be detected is set to an ion such as its m/z.
In the cases of the TOF type and ion trap type, the scan mode may be used, and, in such cases, the mass range is preferably set to 400 to 4000. The marker sugar chain is detected in the above-mentioned analysis conditions at the retention time and m/z below, but these may vary depending on the column and the eluent selected, and hence are not restricted. The ion to be detected is not restricted to the parent ion, and may be an associated ion such as a fragment ion, loaded ion or dimeric ion.
In cases where the detection/measurement is carried out using liquid chromatography, the specification of the liquid chromatography is not restricted as long as the liquid chromatography can stably send a liquid. More particularly, for example, normal-phase HPLC is carried out using a column such as Asahipak NH2P-50 4.6 mm I.D. x 250 mm (manufactured by shodex) at a flow rate of 0.6 ml/min. at a column temperature of 30°C. In this operation, Solvent A, which is a solution of 93% acetonitrile and 0.009% acetic acid, whose pH was adjusted to 6.8 with 25% aqueous ammonia; and Solvent B, which is a solution of 20% acetonitrile and 0.009% acetic acid, whose pH was adjusted to 6.8 with 25% aqueous ammonia; are used, and the Solvent A: Solvent B ratio is 75:25 at the beginning. After injection of a sample, the ratio of Solvent B is linearly increased to 42% for 180 minutes. HPLC analysis is performed using Prominence (manufactured by Shimadzu Corporation) and the respective peaks are quantified using LC station (manufactured by Shimadzu Corporation).
Further, a method is preferred wherein the elution times of standard sugar chains (e.g., PA-sugar chains M2A, M3B, M4B, M5A, M6B, M7A, M8A and M9A, manufactured by Takara) are used as internal standards (mannose unit, MU) to calculate MU of the eluted fluorescently labeled sugar chain, and the respective peaks sorted based on the mannose unit are subjected to quantitative analysis of the peak areas between samples, to thereby determine the sugar chain content.

The content of the marker sugar chain detected by the above method or the value calculated based on the amount of the hepatocellular carcinoma marker is used as an index to judge the possibility that the patient who provided the sample is suffering from hepatocellular carcinoma. In the method for diagnosis of hepatocellular carcinoma of the present invention, the content of the marker sugar chain does not need to be determined as an absolute amount, and may be obtained by digitization of the respective peaks unique to the marker sugar chain detected by the above method or the like. Particular examples of the method include a method wherein the height of each detected peak is digitized and a method wherein the peak area is digitized. In liquid chromatography, the method is not restricted to one of these since the method has a quantitative nature, whereas in LC-MS, the method wherein the peak area is digitized is more accurate and preferred. Further, by using a lectin or antibody that specifically recognizes the marker sugar chain of the present invention, the abundance of the marker sugar chain can be measured by a conventional method. The lectin to be used for detecting the substance of the present invention for detection of hepatocellular carcinoma is a substance having a specific binding activity to a bisecting-type sugar chain structure among N-linked sugar chains. The value calculated by digitizing the thus obtained peak may be hereinafter referred to as the "peak strength".

In the method for diagnosis of hepatocellular carcinoma of the present invention, a method using as an index a value calculated based on the amount of the above-mentioned marker sugar chain is preferably used, in addition to the above-described method using as an index the amount of the marker sugar chain. The particular method of calculation may be any method as long as the value to be used as an index in the measurement method of the present invention is corrected such that the value accurately reflects the conditions in the body of the subject, and examples of the method include a method wherein the correction is performed using the amount of the serum to be treated, and a method wherein the correction is performed using the weight of the protein to be treated. Further, a method wherein the amount of a sugar chain that does not fluctuate, or hardly fluctuates, due to the disease, that is, the amount of a sugar chain that does not show a significant difference in the content in the body fluid between healthy individuals and patients suffering from hepatocellular carcinoma (the amount may be hereinafter referred to as the "internal standard") is used as a standard to determine its ratio with respect to the amount of the marker sugar chain is also effective.

As the above-mentioned value calculated based on the amount of the marker sugar chain, in cases where serum is used as the sample, the amount of the marker sugar chain or a value corrected by the internal standard may be used. Examples of internal standard herein include sugar chains showing no significant difference in their contents in the sample between healthy animals and animals suffering from hepatocellular carcinoma.

The hepatocellular carcinoma marker of the present invention shows significant difference in its abundance in the serum between patients suffering from liver cirrhosis and patients suffering from hepatocellular carcinoma, and hence is preferably used to distinguish between these patients in diagnosis. Patients suffering from liver cirrhosis characteristically show extensive tuberculation in the liver, extensive fibrosis, necrosis of hepatocytes, reduction of the blood flow, decreased secretion of bilirubin, jaundice and/or the like. Thus, in the diseased state of liver cirrhosis, complications such as ascites, esophageal varices and/or hepatic encephalopathy are observed. In the present invention, liver cirrhosis is a state wherein, although extensive tuberculation is observed due to infection with HCV, existence of cancer cannot be confirmed by known hepatocellular carcinoma markers and diagnostic imaging (Ultra Sound; US, Computer Tomography; CT, Magnetic Resonance Imaging; MRI), and no complication caused by liver cirrhosis is observed.

The marker sugar chain of the present invention exists in a healthy individual in a small amount but exists in a patient suffering from hepatocellular carcinoma in a remarkably large amount. Therefore, in cases where the content of the hepatocellular carcinoma marker or a value calculated based on the amount of the hepatocellular carcinoma marker is larger than that of a healthy individual, the subject can be diagnosed as highly possibly having developed hepatocellular carcinoma. Further, since the content of the marker sugar chain of the present invention is higher in a patient suffering from hepatocellular carcinoma than in a patient suffering from liver cirrhosis, liver cirrhosis and hepatocellular carcinoma can be distinguished from each other, and diagnosis of hepatocellular carcinoma is thereby possible.
Further, among the sugar chain markers of the present invention, M5A, A2G0B, A2G1(6)FB and A2G2B showed significant difference also between patients suffering from early-stage hepatocellular carcinoma (Stage I according to the TNM classification) and healthy individuals. Thus, by detecting these marker sugar chains, early-stage hepatocellular carcinoma, whose detection has been impossible with the hepatocellular carcinoma markers reported so far, can be diagnosed.
Further, since the content of the sugar chain marker of the present invention has a tendency to increase as the stage of hepatocellular carcinoma advances (Fig. 2), the content of the hepatocellular carcinoma marker of the present invention or a value calculated therefrom may also be used for identifying the stage of progression of hepatocellular carcinoma.
In the present description, the TNM classification (Stages I to IV) means a classification of the stage of progression of hepatocellular carcinoma according to TNM Classification, 6th ed., published in 2002 by Union for International Cancer Control (UICC) (see Japanese Translated PCT Patent Application Laid-open No. 2008-505143).

### <Method for Evaluating Therapeutic or Prophylactic Agent for Hepatocellular Carcinoma>

By administering a prophylactic or therapeutic agent for hepatocellular carcinoma to an animal requiring prophylaxis or treatment of hepatocellular carcinoma and measuring the content of the above hepatocellular carcinoma marker in body fluid collected from the animal, evaluation of the prophylactic or therapeutic effect against hepatocellular carcinoma in the animal can be carried out using as an index the content of the hepatocellular carcinoma marker or a value calculated therefrom.
For example, the content of the hepatocellular carcinoma marker or a value calculated therefrom observed before administration of the prophylactic or therapeutic agent for hepatocellular carcinoma is compared with that observed several days to several months after the administration, and, if the content of the hepatocellular carcinoma marker or the value calculated therefrom is lower in the latter case, the agent can be judged to have had a prophylactic or therapeutic effect.
The animal to be evaluated is preferably human.
Examples of the therapeutic agent for hepatocellular carcinoma include tegafur (general name: uracil), epirubicin (general name), mitomycin C (general name), fluorouracil (general name), cyclophosphamide (general name) and mitoxantrone (general name).

### <Method for Evaluating Candidate Compound for Therapeutic or Prophylactic Agent for Hepatocellular Carcinoma>

Further, by administering a candidate compound for a prophylactic or therapeutic agent for hepatocellular carcinoma to an animal in need of prophylaxis or treatment of hepatocellular carcinoma and subsequently measuring the amount of the above hepatocellular carcinoma marker in body fluid collected from the animal, evaluation of the prophylactic or therapeutic effect of the candidate compound against hepatocellular carcinoma can be carried out using as an index the amount of the hepatocellular carcinoma marker or a value calculated therefrom.
For example, the content of the hepatocellular carcinoma marker or a value calculated therefrom observed before administration of the candidate compound is compared with that observed several days to several months after the administration, and, if the content of the hepatocellular carcinoma marker or the value calculated therefrom is lower in the latter case, the compound can be judged to be a promising candidate substance for a prophylactic or therapeutic agent for hepatocellular carcinoma.
The candidate compound may be either a low molecular weight compound or a peptide or protein. The animal to be evaluated is preferably human.

### <Kit for Diagnosis of Hepatocellular Carcinoma, for Evaluation of Therapeutic Effect against Hepatocellular Carcinoma, or for Evaluation of Prophylactic or Therapeutic Effect of Candidate drug Compound against Hepatocellular Carcinoma>

The kit of the present invention for diagnosis of hepatocellular carcinoma, for evaluation of a therapeutic effect against hepatocellular carcinoma, or for evaluation of a prophylactic or therapeutic effect of a candidate drug compound against hepatocellular carcinoma comprises a reagent with which the amount(s) of one or more of the marker sugar chains of the present invention can be measured. Examples of such a reagent include the above-described sugar chains to be used as standard substances in normal-phase and reversed-phase high-performance liquid chromatography, mass spectrometry, nuclear magnetic resonance and the like; and antibodies and lectins that specifically recognize the marker sugar chain of the present invention. The antibodies that specifically recognize the marker sugar chain of the present invention may be any of polyclonal antibodies and monoclonal antibodies, and fragments thereof, and can be obtained by a known method using the marker sugar chain as an antigen. Examples of the lectins that specifically recognize the marker sugar chain of the present invention include those described above.

### EXAMPLES

The present invention will now be described in more detail by way of Examples shown below, but the present invention is not limited to thereto as long as the gist of the present invention is not impaired.

### Example 1

### Determination of Hepatocellular Carcinoma Marker

### (1) Measurement of Amount of Maker Sugar Chain in Sample Serum

Sera were obtained from patients clinically diagnosed as having hepatocellular carcinoma (hereinafter referred to as hepatocellular carcinoma), patients clinically diagnosed as having liver cirrhosis accompanied by HCV infection (hereinafter referred to as liver cirrhosis), and healthy individuals with no sign of liver diseases (hereinafter referred to as healthy individuals), as subjects. More particularly, glycoprotein sugar chain analysis was carried out using sera of 69 healthy individuals, 7 patients suffering from chronic hepatitis, 8 patients suffering from liver cirrhosis and 55 patients suffering from hepatocellular carcinoma. The 55 patients suffering from hepatocellular carcinoma were classified into 4 stages based on the stage of progression as described in Table 2 (TNM classification). The sera of the 69 healthy individuals were collected after obtaining informed consent in Soiken. The sera of patients suffering from the liver diseases were collected after obtaining informed consent in Faculty of Medicine, Kagawa University. Details of the samples are as shown in Table 2.

**[Table 2]**

| | healthy subj ect | liver cirrhosis | hepatic carcinoma | | | |
|---|---|---|---|---|---|---|
| | | | Stage I | Stage II | Stage III | Stage IV |
| number of subjects | 69 | 8 | 7 | 17 | 18 | 13 |
| mean age | 44.1 | 69.3 | 74.6 | 72.7 | 71.4 | 68.7 |
| age range | 21-65 | 57-77 | 60-85 | 59-82 | 63-83 | 50-80 |
| male/female | 26/45 | 3/5 | 5/2 | 6/8 | 15/3 | 5/8 |

In pretreatment of the samples, 9 volumes of acetone at -20°C was added to each collected serum and the resulting mixture was mixed well. After leaving the mixture to stand for a while, the mixture was centrifuged and the supernatant was then removed. The precipitate was freeze-dried, and anhydrous hydrazine was added to 2 mg dry weight of the sample, followed by decomposition of hydrazine by heating at 100°C for 10 hours, to remove sugar chains from proteins. Subsequently, hydrazine was evaporated, and reacetylated sugar chains were purified by a part of the methods described in JP 2005-308697 A and Anal. Biochem. 384, 324-326, 2006 using a solid-phase-extraction-type graphite carbon column (manufactured by GL Sciences Inc.).

The purified sugar chains were fluorescently labeled by 2-aminopyridyl amination (2-aminopyridine; PA, manufactured by Nacarai tesque, Inc.). To remove unreacted PA, the sample was passed through a solid-phase cellulose column. A solution of 66.7% 1-butanol, 16.7% ethanol and 100 mM ammonium acetate was prepared as a washing solution to be used for the solid-phase cellulose column, and a solution of 33% ethanol and 15 mM ammonium bicarbonate was prepared as an eluent for eluting fluorescently labeled sugar chains. From the obtained pool of fluorescently labeled serum sugar chains, neutral sugar chains existing in the serum were separated by ion-exchange chromatography (DE52, manufactured by Whatman). In terms of the solutions to be used in this operation, a solution prepared by adjusting pH of pure water to 9.0 with 25% aqueous ammonia was used as Solution A, and a solution prepared by adjusting pH of 500 mM aqueous ammonium acetate solution to 9.0 with 25% aqueous ammonia was used as Solution B.

At the same time, the same amount of the pool of serum sugar chains was treated with neuraminidase (*Arthrobacter ureafaciens,* manufactured by Nacarai tesque, Inc.) at 37°C for 12 hours, and ion-exchange chromatography was then performed, to purify only asialo sugar chains produced by removal of sialic acid from their side chains.

The neutral sugar chains and asialo sugar chains that were fluorescently labeled as described above were subjected to normal-phase high-performance liquid chromatography (normal-phase HPLC), and the amounts of sugar chains were measured by a fluorescence detector. For the normal-phase HPLC, a column Asahipak NH2P-50 4.6 mm I.D. x 250mm (manufactured by shodex) was used at a flow rate of 0.6 ml/min. at a column temperature of 30°C. In this operation, Solvent A, which is a solution of 93% acetonitrile and 0.009% acetic acid, whose pH was adjusted to 6.8 with 25% aqueous ammonia; and Solvent B, which is a solution of 20% acetonitrile and 0.009% acetic acid, whose pH was adjusted to 6.8 with 25% aqueous ammonia; were used, and the solvent A: solvent B ratio was 75:25 at the beginning. After injection of each sample, the ratio of Solvent B was linearly increased to 42% for 180 minutes. HPLC analysis was performed using Prominence (manufactured by Shimadzu Corporation) and the respective peaks were quantified using LC station (manufactured by Shimadzu Corporation).

Further, the elution times of the standard sugar chains (PA-sugar chains M2A, M3B, M4B, M5A, M6B, M7A, M8A and M9A, manufactured by Takara) were used as internal standards (mannose unit, MU) to calculate MU of the eluted fluorescently labeled sugar chains. For each peak separated based on the mannose unit, quantitative analysis of the peak area was carried out among the samples.

For identification of the sugar chain structure, commercially available standard sugar chains were subjected to normal-phase HPLC and reversed-phase HPLC, and MU calculated based on the internal standard in the normal-phase HPLC and GU calculated based on the internal standard (glucose unit, GU3-22, manufactured by TaKaRa) in the reversed-phase HPLC were used to obtain a two-dimensional map (Fig. 1).
For the reversed-phase HPLC, a column Develosil C30 (manufactured by Nomura Kagakus) 4.6 mm I.D. x 150 mm was used at a flow rate of 0.5 ml/min. at a column temperature of 30°C. In this operation, Solvent A, which is a solution adjusted to 5 mM ammonium acetate (pH 4.0); and Solvent B, which is a solution adjusted to 10% acetonitrile and 5 mM ammonium acetate (pH 4.0); were used, and the Solvent A: Solvent B ratio was 75:25 at the beginning. After injection of each sample, the ratio of Solvent B was linearly increased to 42% for 60 minutes.

The identified sugar chain structures and abundances of the thus measured/identified N-linked sugar chains in the healthy individuals (A), liver cirrhosis (B) and hepatocellular carcinoma (C) are shown in Table 3. As is evident from the table, the sugar chains shown in Table 3 were present in low abundances in both the sera of the healthy individuals and the sera of liver cirrhosis, while these were present in the sera of hepatocellular carcinoma in very large amounts. Further, in addition to hepatocellular carcinoma and liver cirrhosis, measurement was carried out in the same manner also in patients suffering from hepatitis, liver cirrhosis accompanied by complications, gastric cancer, pancreatic cancer, digestive diseases, adenoma, polyp and central nervous system diseases, and the measured values are plotted in Fig. 2.

**[Table 3]**

| | marker sugar chain | amount ± S D | | |
|---|---|---|---|---|
| | | healthy subject | liver cirrhosis | hepatic carcicoma |
| neutral sugar chain | **M5A** | 6.79 ± **1.88** | 7.24 ± **3.61** | 16.93 ±**4.78** |
| | **A2G0** | 1.18 ± **0.55** | 3.04 ± **2.27** | 7.20 ± **4.27** |
| | **A2G0B** | 2.12 ±**0.68** | 4.34 ± **3.57** | 12.75 ±**6.24** |
| | **A2G1(6)FB** | 3.95 ±**1.50** | 7.05 ±**2.21** | 16.11 ±**6.42** |
| asialo sugar chain | **A2G2B** | 2.04 ±**1.18** | 3.07 ±**0.67** | 7.42 ±**3.78** |
| | **A2G2Fo2** | 1.91 ±**1.07** | 2.84 ±**1.31** | 7.73 ±**5.00** |

### (2) Test of Marker Sugar Chains

For the marker sugar chains for detection of hepatocellular carcinoma, which were determined by the above glycoprotein sugar chain analysis, the values of the sensitivity, specificity and positive predictive value (PPV) are shown. The sensitivity represents the positive rate of each marker in a sample having hepatocellular carcinoma. The specificity represents the negative rate of each marker in a sample having no hepatocellular carcinoma. PPV represents the probability of having hepatocellular carcinoma in cases where each marker is positive. The cut-off value employed herein was set to the value 1.2-fold higher than the mean abundance of each marker observed for the sera of patients suffering from liver cirrhosis.
As shown in Table 4, the neutral sugar chain A2G0B showed a sensitivity of 91%, specificity of 96% and PPV of 96%. Further, the neutral sugar chain A2G1(6)FB showed a sensitivity of 89%, specificity of 96% and PPV of 94%.

**[Table 4]**

| | marker | sensitivity | specificity | PPV |
|---|---|---|---|---|
| neutral sugar chain | M5A | 100% | 82% | 79% |
| | A2G0 | 84% | 98% | 96% |
| | A2G0B | 91% | 96% | 94% |
| | A2G1(6)FB | 8 9 % | 96% | 94% |
| asialo sugar chain | A2G2B | 84% | 88% | 82% |
| | A2G2Fo2 | 84% | 87% | 81% |

On the other hand, as described in J. Gastroenterol. Hepatol. 14. 436-445, 1999 and Cancer Research 53, 5419-5423, 1993, AFP, which is a known hepatocellular carcinoma marker, is reported to have a sensitivity of 70% and specificity of 70% (cut-off value, 20 ng/ml), and AFP-L3 is reported to have a sensitivity of 70%, specificity of 90% and PPV of 77%. Further, it was recently shown that a branch alpha(1,3)-fucosylated triantennary glycan, which is a hepatocellular carcinoma marker and an N-linked sugar chain, increases in the serum of hepatocellular carcinoma patients and hence can be used as a hepatocellular carcinoma marker (Hepatology 46, 1426-1435, 2008). However, this glycan shows a sensitivity of 57%, specificity of 88% and PPV of 81% (limited to HBV-positive liver cirrhosis and hepatocellular carcinoma).

Table 5 shows the results of analysis of significance of fluctuation of each sugar chain marker in terms of comparisons between healthy individuals and liver cirrhosis, between liver cirrhosis and hepatocellular carcinoma, and between liver cirrhosis and hepatocellular carcinoma at various stages of progression, which analysis was carried out using Student t-test (P value). In the table, parentheses indicate control groups, and each sugar chain marker is judged to show a biologically significant difference in cases where the P value is not more than 0.05.

**[Table 5]**

| | | | | hepatic carcinoma (vs liver cirrhosis) | | | |
|---|---|---|---|---|---|---|---|
| | marker | liver cirrhosis (vs healthy) | Total hepatic carcinoma (vs liver cirrhosis) | StageI | StageII | StageIII | StageIV |
| neutral sugar chain | M5A | 0.7432 | <0.0001 | 0.0055 | <0.0001 | <0.0001 | <0.0001 |
| | A2G0 | 0.0604 | 0.0009 | 0.0758 | 0.0059 | 0.0034 | 0.0034 |
| | A2G0B | 0.1332 | <0.0001 | 0.0504 | 0.0001 | 0.0001 | 0.0007 |
| | A2G1(6)FB | 0.0060 | <0.0001 | 0.0066 | <0.0001 | <0.0001 | 0.0003 |
| | A2G2B | 0.7079 | <0.0001 | 0.0081 | <0.0001 | <0.0001 | 0.0051 |
| asialo sugar chain | A2G2B | 0.0048 | <0.0001 | 0.0600 | 0.0001 | <0.0001 | 0.0037 |
| | A2G2Fo2 | 0.1142 | 0.0115 | 0.1020 | 0.0033 | <0.0001 | 0.0011 |

As is evident from Table 5, each of the marker sugar chains M5A, A2G0, A2G0B, A2G1(6)FB and A2G2B, which are neutral sugar chains of the present invention, and asialo A2G2B and asialo A2G2Fo2 showed a significant difference between the values in healthy individuals and patients suffering from hepatocellular carcinoma. Further, A2G1(6)FB and A2G2B, which are marker sugar chains of the present invention, showed a significant difference also between liver cirrhosis and healthy individuals. Therefore, for example, these marker sugar chains may be effectively used for tracing progression of the diseased state from HCV infection to liver cirrhosis and then to hepatocellular carcinoma.

In particular, among the sugar chain markers of the present invention, the neutral sugar chains M5A, A2G0B A2G1(6)FB and A2G2B showed significant difference between patients suffering from early-stage hepatocellular carcinoma (Stage I) and healthy individuals. By detecting the above sugar chain markers, early-stage hepatocellular carcinoma, whose detection has been impossible with the hepatocellular carcinoma markers reported so far, can now be diagnosed.
J. Proteome Res. 8, 595-602, 2009 and JP2008-541060 disclose fucosylated hemopexin as a hepatocellular carcinoma marker. Fucosylated hemopexin is said to have a sensitivity of 92%, specificity of 92% and PPV of 100% as a hepatocellular carcinoma marker. However, whether detection of fucosylated hemopexin enables accurate diagnosis of early-stage hepatocellular carcinoma (Stage I according to the TNM classification) has not been revealed. On the other hand, M5, A2G0B, A2G1(6)FB and A2G2B, which are hepatocellular carcinoma markers of the present invention, show almost the same or higher significance also in a patient group (total hepatocellular carcinoma) equivalent to the one in the above-described documents.

### (3) Comparison with Existing Markers

Table 6 shows the rates of detection of hepatocellular carcinoma with marker sugar chains of the present invention in, among the samples obtained in the above (1) from patients diagnosed as having hepatocellular carcinoma, the samples which are negative for AFP, which is an existing hepatocellular carcinoma marker (< 30 ng/ml) (21/55 samples); the samples negative for PIVKAII, which is similarly an existing hepatocellular carcinoma marker (< 40 mAU/ml) (18/55 samples); and the samples negative for AFP and negative for PIVKAII (11/55 samples) (the cut-off value employed here was set to the value 1.2-fold higher than the mean expression level of each sugar chain marker observed for the sera of patients suffering from liver cirrhosis). The values for AFP and PIVKAII were tested under contract with an outside examination institute. The EIA method, which is commonly used, was employed as the examination method.

**[Table 6]**

| | marker | marker sugar chain positive /AFP negative (%) | marker sugar chain positive /PIVKAII negative (%) | marker sugar chain positive /AFP&PIVKAII negative (%) |
|---|---|---|---|---|
| neutral sugar chain | M5 | 21/21 (100) | 18/18(100) | 11/11 (100) |
| | A2G0 | 19/21 (90) | 17/18 (94) | 11/11 (100) |
| | A2G0B | 19/21 (90) | 17/18 (94) | 10/11 (91) |
| | A2G1(6)FB | 17/21 (81) | 16/18 (89) | 10/11(91) |
| | A2G2B | 20/21 (95) | 18/18(100) | 11/11 (100) |
| asialo sugar chain | A2G2B | 18/21 (86) | 13/18 (72) | 9/11 (82) |
| | A2G2Fo2 | 19/21 (90) | 16/18 (89) | 8/11 (73) |

As is evident from Table 6, A2G0B, among the sugar chain markers of the present invention, succeeded in detection of hepatocellular carcinoma in 90% of the hepatocellular carcinoma samples negative for AFP (19/21 samples), with values not less than the cut-off value. Further, this sugar chain marker also succeeded in detection of hepatocellular carcinoma in 94% of the hepatocellular carcinoma samples negative for PIVKAII (17/18 samples), with values not less than the cut-off value. Further, this sugar chain marker also succeeded in detection of hepatocellular carcinoma in 91% (10/11 samples) of the hepatocellular carcinoma samples negative for both AFP and PIVKAII (11/56 samples), with values not less than the cut-off value. There was no correlation between the expression level of the sugar chain marker in the serum of hepatocellular carcinoma and the values for the existing hepatocellular carcinoma markers AFP and PIVKAII (Figs. 3 and 4).

## Claims

1. A hepatocellular carcinoma marker comprising a sugar chain represented by any one of the (1) to (7) shown below:

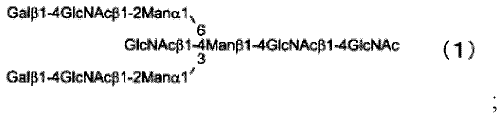

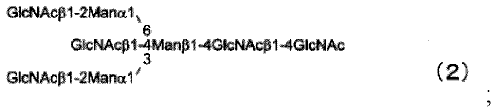

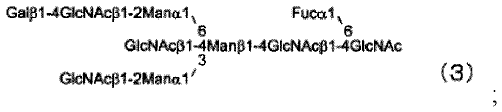

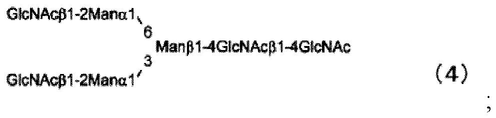

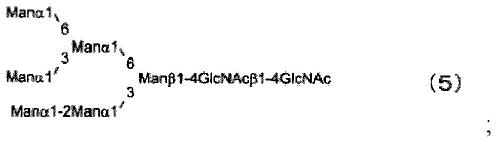

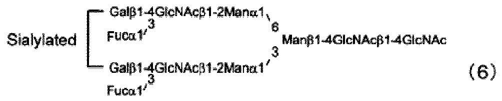

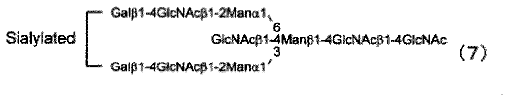

2. A measurement method for diagnosis of hepatocellular carcinoma in a subject animal, comprising measuring the amount of the hepatocellular carcinoma marker according to claim 1 in body fluid collected from said animal, and using the amount of the hepatocellular carcinoma marker or a value calculated based on the amount of the hepatocellular carcinoma marker as an index.

3. The measurement method for diagnosis of hepatocellular carcinoma according to claim 2, wherein said step of measuring the amount of said hepatocellular carcinoma marker according to claim 1 comprises preliminarily extracting a complex of a protein which is bound to the sugar chain as the marker and the sugar chain from the body fluid by using a substance which specifically binds to the protein.

4. A method for evaluating a prophylactic or therapeutic effect against hepatocellular carcinoma in an animal in need of prophylaxis or treatment of hepatocellular carcinoma, comprising
administering a prophylactic or therapeutic agent for hepatocellular carcinoma to said animal,
measuring the amount of said hepatocellular carcinoma marker according to claim 1 in body fluid collected from said animal, and
using the amount of said hepatocellular carcinoma marker or a value calculated based on the amount of said hepatocellular carcinoma marker as an index.

5. The method for evaluating a prophylactic or therapeutic effect against hepatocellular carcinoma according to claim 4, wherein said step of measuring the amount of said hepatocellular carcinoma marker according to claim 1 comprises preliminarily extracting a complex of a protein which is bound to the sugar chain as the marker and the sugar chain from the body fluid by using a substance which specifically binds to the protein.

6. A method for evaluating a prophylactic or therapeutic effect of a candidate drug compound against hepatocellular carcinoma, comprising
administering a candidate drug compound to an animal in need of prophylaxis or treatment of hepatocellular carcinoma,
measuring the amount of said hepatocellular carcinoma marker according to claim 1 in body fluid collected from said animal, and
using the amount of said hepatocellular carcinoma marker or a value calculated based on the amount of said hepatocellular carcinoma marker as an index.

7. The method for evaluating a prophylactic or therapeutic effect of a candidate drug compound according to claim 6, wherein said step of measuring the amount of said hepatocellular carcinoma marker according to claim 1 comprises preliminarily extracting a complex of a protein which is bound to the sugar chain as the marker and the sugar chain from the body fluid by using a substance which specifically binds to the protein.

8. The method according to any of claims 2 to 7, wherein said value calculated based on the amount of said hepatocellular carcinoma marker is a value calculated using the ratio between the amounts of: a sugar chain showing no significant difference in the content between the samples from healthy animals and the samples from animals suffering from hepatocellular carcinoma; and said hepatocellular carcinoma marker.

9. A kit for diagnosis of hepatocellular carcinoma, for evaluation of a therapeutic effect against hepatocellular carcinoma, or for evaluation of a prophylactic or therapeutic effect of a candidate drug compound against hepatocellular carcinoma, said kit comprising a reagent with which the amount of any of said hepatocellular carcinoma markers according to claim 1 can be measured.
